# EUROPEAN PATENT SPECIFICATION

(11) **EP 2 679 256 B1**
(45) Date of publication and mention of the grant of the patent: **15.04.2020**
(21) Application number: 11859626.1
(22) Date of filing: 20.05.2011
(51) Int. Cl.: A61B 5/026, A61B 5/021, A61M 1/36

(54) **BLOOD PRESSURE DECREASE PREDICTION APPARATUS**
VORRICHTUNG ZUR VORHERSAGE EINES BLUTDRUCKABFALLS
DISPOSITIF DE PRÉDICTION DE DIMINUTION DE PRESSION ARTÉRIELLE

(30) Priority: 25.02.2011 WO PCT/JP2011/054347
(43) Date of publication of application: 01.01.2014
(73) Proprietor: Pioneer Corporation, Tokyo 113-0021 (JP)
(72) Inventor: GOMA, Masaki, Kawasaki-shi Kanagawa 212-0031 (JP); KIMURA, Yoshinori, Kawasaki-shi Kanagawa 212-0031 (JP); ITO, Atsuya, Kawasaki-shi Kanagawa 212-0031 (JP); MORI, Shuntaro, Kawasaki-shi Kanagawa 212-0031 (JP)
(74) Representative: Viering, Jentschura & Partner mbB Patent- und Rechtsanwälte
(86) International application number: PCT/JP2011/061615
(87) International publication number: WO 2012/114544

(56) References cited:
- EP-A1- 0 956 815
- JP-A- 11 221 275
- JP-A- 2000 135 202
- JP-A- 2002 065 847
- JP-A- 2003 010 319
- JP-A- 2004 248 793
- JP-A- 2004 357 784
- JP-A- 2007 143 815
- US-A1- 2004 254 473
- US-A1- 2009 082 684
- JUN NIWAYAMA ET AL: "Prediction method for decreases in blood pressure during hemocatharsis therapy by arteriolar blood flow measurement", JOURNAL OF ARTIFICIAL ORGANS ; THE OFFICIAL JOURNAL OF THE JAPANESE SOCIETY FOR ARTIFICIAL ORGANS, SPRINGER-VERLAG, TO, vol. 10, no. 1, 23 March 2007 (2007-03-23) , pages 36-41, XP019475299, ISSN: 1619-0904, DOI: 10.1007/S10047-006-0357-9

## Description

### Technical Field

The present invention relates to a blood pressure decrease prediction apparatus which predicts a decrease of a blood pressure of a living body (a biological object, an organism).

### Background Art

There is a possibility of a decrease of a patient's blood pressure during an artificial dialysis, because of a decrease of a circulating blood volume due to removal of water. If the blood pressure is decreased, the patient may be in a state of shock. So, patent documents 1 and 2 propose the technology which is to promptly measure the blood pressure of the patient on the artificial dialysis. For example, the patent document 1 discloses a technology which continuously monitors the blood pressure of the patient on a dialysis therapy by continuously calculating a correlation value of the blood pressure based on a difference between a phase of heart electronic potential (electronic potential of a heart) and a phase of volume pulse wave (PTG: PleThysmoGram). For example, the patent document 2 discloses a technology which detects the decrease of the blood pressure by dividing a period for a hemodialysis into a plurality of periods for each patient and measuring the blood pressure in each of the plurality of the divided periods with a cuff.

On the other hand, a non patent document 1 discloses a technology which predicts the decrease of the blood pressure by monitoring variation of a blood flow volume (a volume of the blood flow) at a head. Incidentally, a non patent document 2 suggests that there is a correlation between the blood flow volume at the head and an average arterial blood pressure if the average arterial blood pressure is equal to or less than 60 mmHG or is equal to or more than 120 mmHG.

US 2004/254473 A discloses a laser blood-flow meter including a laser-beam irradiator for irradiating laser beams to biological structure, a detector for detecting scattered beams resulted from scattering of the laser beams in the biological structure, the laser blood-flow meter measuring blood flow of the biological structure in accordance with the scattered beams detected by the detector, and a beam-collector for collecting the scattered beams to direct the collected beams to the detector.

### Background Art Document

### Patent Document

Patent document 1: Japanese Patent Application Laid Open No. Hei05-285218
Patent document 2: Japanese Patent Application Laid Open No. 2000-000217

### Non Patent Document

Non Patent Document 1: Jun Niwayama et al. Prediction method for decreases in blood pressure during hemocatharsis therapy by arteriolar blood flow measurement. Journal of Artificial Organs Volume 10, Number 1, 36 to 41
Non Patent Document 2: LASSEN NA. Cerebral blood flow and oxygen consumption in man. Physiol Rev. 1959 Apr: 39(2): 183-238
Non Patent Document 3: Yoshiharu Tsubakihara. How to improve the continuous low blood pressure of the patient who receives dialysis for long period ? Dialysis Care 8(12): 19 to 24. 2002
Non Patent Document 4: Takahiro Niizato. Abnormal blood pressure which occurs during the hemodialysis. Clinical Engineering 13(10) 2002

### Disclosure of Invention

### Subject to be Solved by the Invention

However, according to the technology disclosed in the above described patent document 1, there is a technical problem which may causes large burden on the patient, for example, may give unfree feeling to the patient, because the heart electronic potential is detected while an electrode (a heart electrical potential measurement sensor) is attached to the patient. Moreover, according to the technology disclosed in the above described patent document 2, there is a technical problem which may causes large burden on the patient due to a pressure (a press) of the cuff, because the blood pressure is repeatedly measured with the cuff.

Furthermore, according to the technology disclosed in the above described patent documents 1 and 2, the blood pressure is already decreased when the decrease of the blood pressure is detected, because the decrease of the blood pressure is detected by continuously measuring the blood pressure. Thus, there is such a technical problem that it is difficult to predict the decrease of the blood pressure. The decrease of the blood pressure may cause the delay of the treatment for the patient. Moreover, according to the technology disclosed in the above described non patent document 1, the blood pressure is already decreased when the decrease of the blood pressure is detected, because the decrease of the blood pressure is detected based on only the blood flow volume at a head. Thus, there is such a technical problem that it is difficult to actually predict the decrease of the blood pressure.

In view of the aforementioned problem, it is therefore an object of the present invention to provide, for example, a blood pressure decrease prediction apparatus which is configured to precociously predict the decrease of the blood pressure of the living body without causing the burden on the living body.

### Means for Solving the Subject

In order to solve the above object, a blood pressure decrease prediction apparatus according to claim 1 is provided.

A blood pressure decrease prediction apparatus of the present disclosure is a blood pressure decrease prediction apparatus which is configured to predict a decrease of a blood pressure of a living body, and the blood pressure decrease prediction apparatus is provided with: a pulse rate calculating device which is configured to calculate a pulse rate of the living body, based on a blood flow waveform which represents a temporal variation of a blood flow volume of the living body; a blood flow volume calculating device which is configured to calculate the blood flow volume of the living body, based on the blood flow waveform; and a blood pressure decrease predicting device which is configured to predict the decrease of the blood pressure, based on a variation of each of the calculated pulse rate and the calculated blood flow volume.

These operation and other advantages of the present invention will become more apparent from the embodiment explained below.

### Brief Description of Drawings

[FIG. 1] FIG. 1 is a block diagram illustrating a configuration of a blood pressure decrease prediction apparatus in a first example.
[FIG. 2] FIG. 2 is an explanatory diagram for explaining a method of calculating pulse rate, blood flow volume and pulse wave amplitude, based on blood flow waveform in the first example.
[FIG. 3] FIG. 3 is a flowchart illustrating a flow of pulse rate calculation process in the first example.
[FIG. 4] FIG. 4 is a flowchart illustrating a flow of blood flow volume calculation process in the first example.
[FIG. 5] FIG. 5 is a flowchart illustrating a flow of pulse wave amplitude calculation process in the first example.
[FIG. 6] FIG. 6 is a flowchart illustrating a flow of determination process of blood pressure decrease risk level in the first example.
[FIG. 7] FIG. 7 is a conceptual diagram conceptually illustrating a variation pattern table in the first example.
[FIG. 8] FIG. 8 is a graph illustrating one example of a temporal variation of the pulse rate, the blood flow volume and the pulse wave amplitude of a living body (a patient) during an artificial dialysis.
[FIG. 9] FIG. 9 is a conceptual diagram conceptually illustrating a variation pattern table in a modified example.

### Mode for Carrying Out the Invention

Hereinafter, an embodiment of the present disclosure will be described.

In order to solve the above subject, a blood pressure decrease prediction apparatus of a first embodiment is a blood pressure decrease prediction apparatus which is configured to predict a decrease of a blood pressure of a living body, and the blood pressure decrease prediction apparatus is provided with: a pulse rate calculating device which is configured to calculate a pulse rate of the living body, based on a blood flow waveform which represents a temporal variation of a blood flow volume of the living body; a blood flow volume calculating device which is configured to calculate the blood flow volume of the living body, based on the blood flow waveform; and a blood pressure decrease predicting device which is configured to predict the decrease of the blood pressure, based on a variation of each of the calculated pulse rate and the calculated blood flow volume.

According to the blood pressure decrease prediction apparatus of the present embodiment, when the blood pressure decrease prediction apparatus operates, the blood flow waveform (i.e. waveform signal which represents the temporal variation of the blood flow volume) of the living body who is an analyte or a patient is inputted from a laser blood flow meter which uses LDF (Laser Doppler Flowmetry) method, for example, and the pulse rate and the blood flow volume of the living body are calculated by the pulse rate calculating device and the blood flow volume calculating device, respectively, based on the inputted blood flow waveform. The pulse rate calculating device typically calculates, as the pulse rate, an oscillation frequency (i.e. an inverse number of a cycle) corresponding to pulse wave of the blood flow waveform. The blood flow volume calculating device typically calculates, as the blood flow volume, an average value of the blood flow waveform during a predetermined time period.

Particularly in the present invention, the blood pressure decrease predicting device predicts the decrease of the blood pressure of the living body, based on the variation of each of the calculated pulse rate and the calculated blood flow volume. For example, the blood pressure decrease predicting device performs a process of determining a risk level which indicates a risk for an occurrence of the decrease of the blood pressure (i.e. a possibility for the occurrence of the decrease of the blood pressure), as a process of predicting the decrease of the blood pressure of the living body. For example, the blood pressure decrease predicting device determines the risk level (i.e. predicts the decrease of the blood pressure) by referring to a predetermined risk level determination table in which a combination of the variation of the pulse rate and the variation of the blood flow volume is associated with the risk level. Specifically, for example, the blood pressure decrease predicting device determines that the risk level is "low" which indicates that the risk is low, if the pulse rate increases and the blood flow volume does not vary. For example, the blood pressure decrease predicting device determines that the risk level is "middle" which indicates that the risk is higher than the "low", if the pulse rate increases and the blood flow volume decreases. For example, the blood pressure decrease predicting device determines that the risk level is "high" which indicates that the risk is higher than the "middle", if both of the pulse rate and the blood flow volume decrease.

Here, the blood pressure is determined based on the product of a beat volume (cardiac output, heart output) and a peripheral vascular resistance (PVR). The "beat volume" is a volume of the blood pumped (transferred) from the heart every one minute and varies depending on a heart rate (a cardiac rate) which varies due to an operation of an autonomic nervous system (a sympathetic nervous / parasympathetic nervous system). The beat volume is determined based on the product of a one-time beat volume, which is a volume of the blood pumped from the heart every one pumping, and the heart rate. Therefore, the blood pressure is determined based on the product of the heart rate, the one-time beat volume and the peripheral vascular resistance (see the non patent document 3). The "peripheral vascular resistance" is a resistance to the flow of the blood in the peripheral artery and varies due to the operation of the autonomic nervous system (especially, the sympathetic nervous system). If the living body is a healthy people, usually, the living body automatically adjusts the heart rate and the peripheral vascular resistance in order to maintain the blood pressure. In other words, the living body maintains the blood pressure by automatically adjusting the heart rate and the peripheral vascular resistance, if the blood pressure almost decreases (see the non patent document 4). Therefore, the heart rate and the peripheral vascular resistance often vary before the blood pressure decreases.

As described above, the blood pressure decrease predicting device predicts the decrease of the blood pressure of the living body based on the variation of each of the calculated pulse rate and the calculated blood flow volume. It is considered that the pulse rate corresponds to the heart rate and the blood flow volume often varies depending on the variation of the peripheral vascular resistance. Thus, the blood pressure decrease predicting device can predict the decrease of the blood pressure (for example, appropriately determine the risk level which indicates the risk for the occurrence of the decrease of the blood pressure) precociously before the decrease of the blood pressure occurs. Therefore, it is possible to inform the living body who is the analyte or the patient and an involved person who is a doctor or a nurse of the risk of the decrease of the blood pressure, by outwardly outputting a result of the prediction (for example, the risk level which indicates the risk for the occurrence of the decrease of the blood pressure) by the blood pressure decrease predicting device as color(s), number(s), character(s), sentence(s), diagram(s), sign(s), sound or the like for example. As a result, it is possible to reduce or prevent a delay of a treatment for the living body whose blood pressure is predicted to decrease (i.e. it is possible to precociously treat the living body who has the risk of the decrease of the blood pressure).

Furthermore, especially in the present invention, there is such an advantageous effect that it is possible to mostly or completely eliminate the burden on the living body, in comparison with the case where the blood pressure is measured by using the cuff or the electrode for example, because the pulse rate and the blood flow volume are calculated based on the blood flow waveform inputted from the laser blood flow meter for example.

As described above, according to the blood pressure decrease prediction apparatus of the present invention, it is possible to precociously predict the decrease of the blood pressure of the living body without causing the burden on the living body

In one aspect of the blood pressure decrease prediction apparatus of the first embodiment, the blood pressure decrease predicting device is provided with a risk level determining device which is configured to determine a risk level which indicates a risk for an occurrence of the decrease of the blood pressure, based on the variation of each of the calculated pulse rate and the calculated blood flow volume.

According to this aspect, the prediction of the decrease of the blood pressure performed by the blood pressure decrease predicting device is realized by the determination of the risk level by the risk level determining device. For example, the risk level determining device determines the risk level by referring to the predetermined risk level determination table in which the combination of the variation of the pulse rate and the variation of the blood flow volume is associated with the risk level. Therefore, it is possible to precociously predict the decrease of the blood pressure of the living body.

In an aspect of the above blood pressure decrease prediction apparatus in which the blood pressure decrease predicting device may be provided with the risk level determining device, the risk level determining device may determine the risk level by referring to a predetermined risk level determination table in which a combination of the variation of the pulse rate and the variation of the blood flow volume is associated with the risk level.

According to this aspect, the risk level determining device determines the risk level which indicates the risk for the occurrence of the decrease of the blood pressure by referring to the predetermined risk level determination table. The risk level determination table is a predetermined look-up table which is for determining the risk level and in which the combination of the variation of the pulse rate and the variation of the blood flow volume is associated with the risk level in the case where the respective combination occurs. For example, in the risk level determination table, the risk level "low" is stored in association with the combination of the increase of the pulse rate and no-variation of the blood flow volume, the risk level "middle" is stored in association with the combination of the increase of the pulse rate and the decrease of the blood flow volume, and the risk level "high" is stored in association with the combination of the decrease of both of the pulse rate and the blood flow volume. Incidentally, the risk level determination table can be prepared by presuming, experimentally, experientially or by a simulation in advance, the risk for the occurrence of the decrease of the blood pressure in the case where the combination of the variation of the pulse rate and the variation of the blood flow volume occurs. When the combination of the variation of the calculated pulse rate and the variation of the calculated blood flow volume matches the combination of the variation of the pulse rate and the variation of the blood flow volume stored in the risk level determination table, the risk level determining device determines that the risk level is one that is associated with the matched combination. Incidentally, when the combination of the variation of the calculated pulse rate and the variation of the calculated blood flow volume does not match all of the combination of the variation of the pulse rate and the variation of the blood flow volume stored in the risk level determination table, it may be determined that the risk level is "none" which indicates that the risk is very few or very low. Therefore, according to this aspect, it is possible to precociously and more certainly predict the decrease of the blood pressure of the living body and to appropriately determine the risk level which indicates the risk for the occurrence of the decrease of the blood pressure.

In another aspect of the blood pressure decrease prediction apparatus of the first embodiment, the blood pressure decrease prediction apparatus is further provided with a pulse wave amplitude calculating device which is configured to calculate a pulse wave amplitude of the living body, based on the blood flow waveform, the blood pressure decrease predicting device predicts the decrease of the blood pressure, based on a variation of the calculated pulse wave amplitude in addition to the variation of each of the calculated pulse rate and the calculated blood flow volume.

According to this aspect, it is possible to more precociously and more appropriately predict the decrease of the blood pressure, because the blood pressure decrease predicting device predicts the decrease of the blood pressure of the living body based on the variation of the calculated pulse wave amplitude in addition to the variation of each of the calculated pulse rate and the calculated blood flow volume.

In an aspect of the blood pressure decrease prediction apparatus which is further provided with the pulse wave amplitude calculating device, the blood pressure decrease predicting device may be provided with a risk level determining device which is configured to determine a risk level which indicates a risk for an occurrence of the decrease of the blood pressure, based on the variation of each of the calculated pulse rate, the calculated blood flow volume and the calculated pulse wave amplitude.

According to this aspect, the prediction of the decrease of the blood pressure performed by the blood pressure decrease predicting device is realized by the determination of the risk level by the risk level determining device. For example, the risk level determining device determines the risk level by referring to a predetermined risk level determination table in which a combination of the variation of the pulse rate, the variation of the blood flow volume and the variation of the pulse wave amplitude is associated with the risk level. Therefore, it is possible to precociously and appropriately predict the decrease of the blood pressure of the living body while segmenting the risk level more finely.

Furthermore, in this case, the risk level determining device may determine the risk level by referring to a predetermined risk level determination table in which a combination of the variation of the pulse rate, the variation of the blood flow volume and the variation of the pulse wave amplitude is associated with the risk level.

According to this aspect, the risk level determining device determines the risk level which indicates the risk for the occurrence of the decrease of the blood pressure by referring to the predetermined risk level determination table. The risk level determination table is a predetermined look-up table which is for determining the risk level and in which the combination of the variation of the pulse rate, the variation of the blood flow volume and the variation of the pulse wave amplitude is associated with the risk level in the case where the respective combination occurs. For example, in the risk level determination table, the risk level "low" is stored in association with the combination of the increase of the pulse rate, no-variation of the blood flow volume and the decrease of the pulse wave amplitude, the risk level "middle" is stored in association with the combination of the increase of the pulse rate, the decrease of the blood flow volume and the decrease of the pulse wave amplitude, and the risk level "high" is stored in association with the combination of the decrease of all of the pulse rate, the blood flow volume and the pulse wave amplitude. Incidentally, the risk level determination table can be prepared by presuming, experimentally, experientially or by a simulation in advance, the risk for the occurrence of the decrease of the blood pressure in the case where the combination of the variation of the pulse rate, the variation of the blood flow volume and the variation of the pulse wave amplitude occurs. When the combination of the variation of the calculated pulse rate, the variation of the calculated blood flow volume and the variation of the calculated pulse wave amplitude matches the combination of the variation of the pulse rate, the variation of the blood flow volume and the variation of the pulse wave amplitude stored in the risk level determination table, the risk level determining device determines that the risk level is one that is associated with the matched combination. Incidentally, when the combination of the variation of the calculated pulse rate, the variation of the calculated blood flow volume and the variation of the calculated pulse wave amplitude does not match all of the combination of the variation of the pulse rate, the variation of the blood flow volume and the variation of the pulse wave amplitude stored in the risk level determination table, it may be determined that the risk level is "none" which indicates that the risk is very few or very low. Therefore, according to this aspect, it is possible to precociously and more certainly predict the decrease of the blood pressure of the living body and to appropriately determine the risk level which indicates the risk for the occurrence of the decrease of the blood pressure because of the segmentation of the risk level.

In an another aspect of the blood pressure decrease prediction apparatus of the first embodiment, the blood pressure decrease predicting device determines whether or not one of the calculated pulse rate and the calculated blood flow volume changes, and predicts the decrease of the blood pressure based on the result of the determination.

According to this aspect, it is possible to precociously and more appropriately predict the decrease of the blood pressure of the living body based on the variation of each of the pulse rate and the blood flow volume of the living body.

In an aspect of the blood pressure decrease prediction apparatus which is further provided with the pulse wave amplitude calculating device, the blood pressure decrease predicting device determines whether or not one of the calculated pulse rate, the calculated blood flow volume and the calculated pulse wave amplitude changes, and predicts the decrease of the blood pressure based on the result of the determination.

According to this aspect, it is possible to precociously and more appropriately predict the decrease of the blood pressure of the living body based on the variation of each of the pulse rate, the blood flow volume and the pulse wave amplitude of the living body.

In an aspect of the blood pressure decrease prediction apparatus in which the blood pressure decrease predicting device is further provided with the risk level determining device, the blood pressure decrease prediction apparatus may be further provided with an outputting device which is configured to outwardly output the risk level determined by the risk level determining device.

According to this aspect, the outputting device outwardly outputs the risk level determined by the risk level determining device with using color(s), number(s), character(s), sentence(s), diagram(s), sign(s), sound or the like for example. Therefore, it is possible to inform the living body who is the analyte or the patient and the involved person who is the doctor or the nurse of the risk of the decrease of the blood pressure. As a result, it is possible to reduce or prevent the delay of the treatment for the living body whose blood pressure is predicted to decrease (i.e. it is possible to precociously treat the living body who has the risk of the decrease of the blood pressure).

These operations and other advantages of the present disclosure will become more apparent from the examples explained below.

### Examples

Hereinafter, examples of the driving apparatus of the present disclosure will be described with reference to the drawings.

### <First Example>

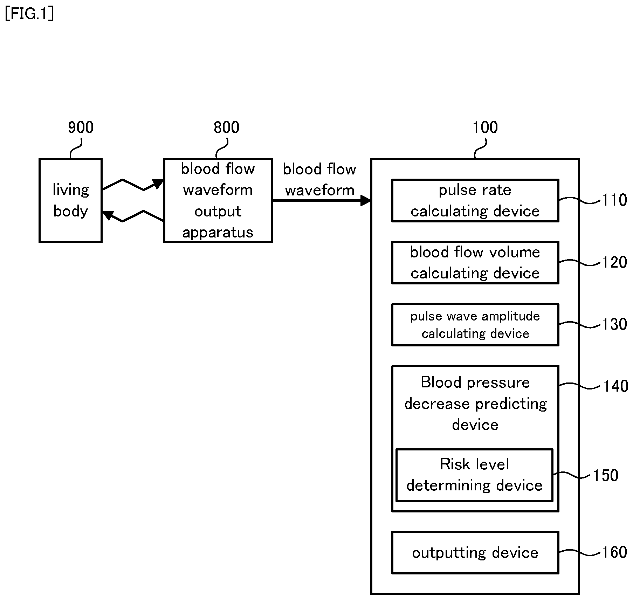

A blood pressure decrease prediction apparatus in a first example will be explained with reference to FIG. 1 to FIG. 8.

Firstly, a configuration of the blood pressure decrease prediction apparatus in the first example will be explained with reference to FIG. 1.

FIG. 1 is a block diagram illustrating the configuration of the blood pressure decrease prediction apparatus in the first example.

In FIG. 1, the blood pressure decrease prediction apparatus 100 in the first example is an apparatus for predicting a decrease of a blood pressure of a living body 900 while the living body 900 who is a patient receives an artificial dialysis, and is provided with a pulse rate calculating device 110, a blood flow volume calculating device 120, a pulse wave amplitude calculating device 130, a blood pressure decrease predicting device 140 which includes a risk level determining device 150, and the outputting device 160. The blood pressure decrease prediction apparatus 100 is configured such that a blood flow waveform which is outputted from a blood flow waveform output apparatus 800 is inputted. The blood flow waveform output apparatus 800 is a laser blood flow meter using LDF (Laser Doppler Flowmetry) method for example, and outputs the blood flow waveform of the living body 900 (i.e. a waveform signal which represents a temporal variation of a blood flow volume).

The pulse rate calculating device 110, the blood flow volume calculating device 120 and the pulse wave amplitude calculating device 130 calculate a pulse rate, the blood flow volume and a pulse wave amplitude of the living body 900, respectively, based on the blood flow waveform inputted from the blood flow waveform output apparatus 800.

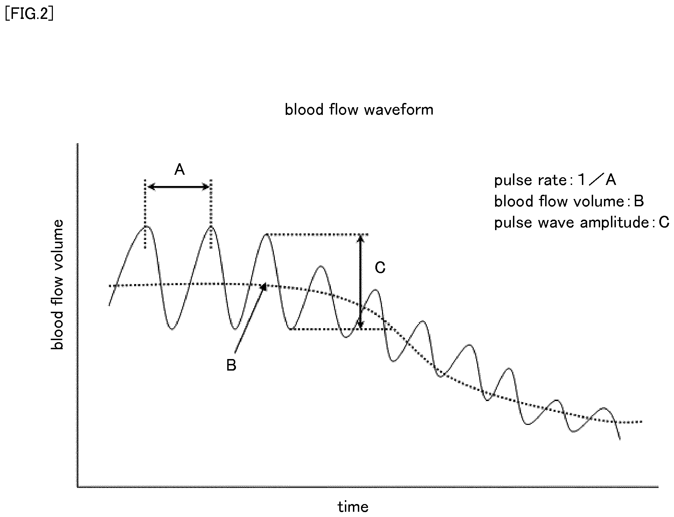

FIG. 2 is an explanatory diagram for explaining a method of calculating the pulse rate, the blood flow volume and the pulse wave amplitude, based on the blood flow waveform in the first example. Incidentally, in FIG. 2, one example of the blood flow waveform which is outputted from the blood flow waveform output apparatus 800 is illustrated.

In FIG. 2, the pulse rate calculating device 110 calculates, as the pulse rate, an oscillation frequency of a waveform corresponding to pulse wave of the blood flow waveform, i.e. an inverse number (1/A) of a cycle A of the blood flow waveform. Incidentally, another calculation method such as a Fast Fourier Transform (FFT) may be used as a method of calculating the pulse rate.

The blood flow volume calculating device 120 calculates, as the blood flow volume, an average value B of the blood flow waveform during a predetermined time period.

The pulse wave amplitude calculating device 130 calculates, as the pulse wave amplitude, an amplitude C of the waveform corresponding to the pulse wave of the blood flow waveform. Incidentally, another calculation method such as a Fast Fourier Transform (FFT) may be used as a method of calculating the pulse wave amplitude.

As described above, the blood pressure decrease prediction apparatus 100 is configured to obtain three variable parameters such as the pulse rate, the blood flow volume and the pulse wave amplitude, from the blood flow waveform which is outputted from the blood flow waveform output apparatus 800.

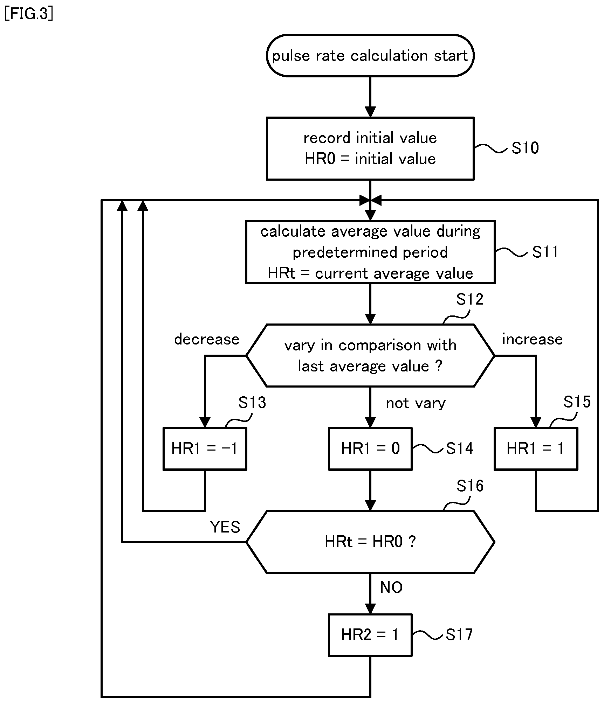

Here, a pulse rate calculation process which is performed by the pulse rate calculating device 110, a blood flow volume calculation process which is performed by the blood flow volume calculating device 120 and a pulse wave amplitude calculation process which is performed by the pulse wave amplitude calculating device 130 will be explained with reference to FIG. 3, FIG. 4 and FIG. 5.

FIG. 3 is a flowchart illustrating a flow of the pulse rate calculation process in the blood pressure decrease prediction apparatus 100.

In FIG. 3, in the pulse rate calculation process, firstly, an initial value of the pulse rate is recorded by the pulse rate calculating device 110 (step S10). Namely, the pulse rate calculating device 110 calculates the pulse rate based on the blood flow waveform which is inputted from the blood flow waveform output apparatus 800 when the artificial dialysis starts, and records, as the initial value HR0 of the pulse rate, the calculated pulse rate into a memory for example.

Next, a current average value HRt of the pulse rate is calculated by the pulse rate calculating device 110 (step S11). Namely, the pulse rate calculating device 110 calculates the average value of the pulse rate during a predetermined period including a current time point based on the blood flow waveform which is inputted from the blood flow waveform output apparatus 800, and records, as the current average value HRt, the calculated average value into the memory for example.

Next, it is determined by the pulse rate calculating device 110 whether or not the current average value HRt varies in comparison with the last average value, more specifically, whether or not the current average value HRt "decreases", "increases" or "does not vary" in comparison with the last average value (step S12). Namely, the pulse rate calculating device 110 calculates the average value HRt of the pulse rate for each predetermined period, determines that the pulse rate decreases if the current average value HRt is smaller than the last average value HRt, determines that the pulse rate increases if the current average value HRt is larger than the last average value HRt, and determines that the pulse rate does not vary if the current average value HRt is same as the last average value HRt.

If it is determined that the pulse rate decreases (step S12: "decrease"), a value of a pulse rate variation flag HR1 is set to "-1" (step S13), if it is determined that the pulse rate does not vary (step S12: "not vary"), the value of the pulse rate variation flag HR1 is set to "0" (step S14), and if it is determined that the pulse rate increases (step S12: "increase"), the value of the pulse rate variation flag HR1 is set to "1" (step S15). Incidentally, the pulse rate variation flag HR1 is a storing device for storing a determination result obtained by determining that the current average value of the pulse rate "decreases", "increases" or "does not vary" in comparison with the last average value.

After the value of the pulse rate variation flag HR1 is set to "-1" (step S13), the process in the step S11 is performed again.

After the value of the pulse rate variation flag HR1 is set to "1" (step S15), the process in the step S11 is performed again.

After the value of the pulse rate variation flag HR1 is set to "0" (step S14), it is determined by the pulse rate calculating device 110 whether or not the current average value HRt is same as the initial value HR0 (step S16). Namely, the pulse rate calculating device 110 determines whether or not the average value HRt which is calculated as the current average value of the pulse rate of the living body 900 is same as the initial value HR0 which is calculated as the pulse rate when the artificial dialysis starts, namely, whether or not the pulse rate of the living body 900 does not vary after the artificial dialysis starts and remains in the initial value HR0. In other words, in the case where the pulse rate does not vary, the pulse rate calculating device 110 determines whether the pulse rate remains in the initial value HR0 which is a value when the artificial dialysis starts or whether the pulse rate varies after the artificial dialysis starts and remains in a value which is different from the initial value HR0.

If it is determined that the current average value HRt is same as the initial value HR0 (step S16: YES), the process in the step S11 is performed again.

If it is determined that the current average value HRt is not same as (i.e. is different from) the initial value HR0 (step S16: NO), a value of a pulse rate saturation flag HR2 is set to "1" (step S17). Incidentally, the pulse rate saturation flag HR2 is a storing device for storing a determination result obtained by determining whether or not the current average value HRt is same as the initial value HR0, and an initial value of the pulse rate saturation flag HR2 is set to "0" which indicates that the current average value HRt is same as the initial value HR0.

As described above, in the pulse rate calculation process, the pulse rate (specifically, the average value of the pulse rate during the predetermined period) is calculated for each predetermined period based on the blood flow waveform which is outputted from the blood flow waveform output apparatus 800 after the artificial dialysis starts, it is determined whether the calculated pulse rate varies and the determination result is recorded into the pulse rate variation flag HR1 and the pulse rate saturation flag HR2.

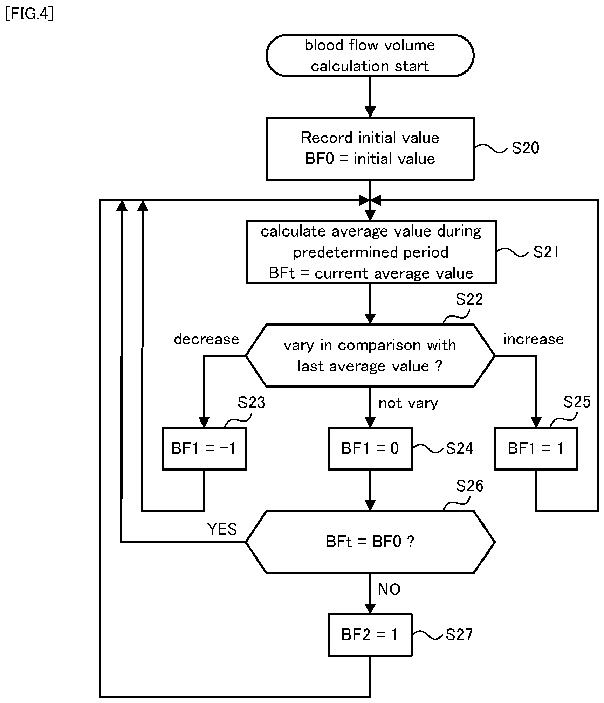

FIG. 4 is a flowchart illustrating a flow of the blood flow volume calculation process in the blood pressure decrease prediction apparatus 100.

In FIG. 4, in the blood flow volume calculation process, firstly, an initial value of the blood flow volume is recorded by the blood flow volume calculating device 120 (step S20). Namely, the blood flow volume calculating device 120 calculates the blood flow volume based on the blood flow waveform which is inputted from the blood flow waveform output apparatus 800 when the artificial dialysis starts, and records, as the initial value BF0 of the blood flow volume, the calculated blood flow volume into a memory for example.

Next, a current average value BFt of the blood flow volume is calculated by the blood flow volume calculating device 120 (step S21). Namely, the blood flow volume calculating device 120 calculates the average value of the blood flow volume during a predetermined period including a current time point based on the blood flow waveform which is inputted from the blood flow waveform output apparatus 800, and records, as the current average value BFt, the calculated average value into the memory for example.

Next, it is determined by the blood flow volume calculating device 120 whether or not the current average value BFt varies in comparison with the last average value, more specifically, whether or not the current average value BFt "decreases", "increases" or "does not vary" in comparison with the last average value (step S22). Namely, the blood flow volume calculating device 120 calculates the average value BFt of the blood flow volume for each predetermined period, determines that the blood flow volume decreases if the current average value BFt is smaller than the last average value BFt, determines that the blood flow volume increases if the current average value BFt is larger than the last average value BFt, and determines that the blood flow volume does not vary if the current average value BFt is same as the last average value BFt.

If it is determined that the blood flow volume decreases (step S22: "decrease"), a value of a blood flow volume variation flag BF1 is set to "-1" (step S23), if it is determined that the blood flow volume does not vary (step S22: "not vary"), the value of the blood flow volume variation flag BF1 is set to "0" (step S24), and if it is determined that the blood flow volume increases (step S22: "increase"), the value of the blood flow volume variation flag BF1 is set to "1" (step S25). Incidentally, the blood flow volume variation flag BF1 is a storing device for storing a determination result obtained by determining that the current average value of the blood flow volume "decreases", "increases" or "does not vary" in comparison with the last average value.

After the value of the blood flow volume variation flag BF1 is set to "-1" (step S23), the process in the step S21 is performed again.

After the value of the blood flow volume variation flag BF1 is set to "1" (step S25), the process in the step S21 is performed again.

After the value of the blood flow volume variation flag BF1 is set to "0" (step S24), it is determined by the blood flow volume calculating device 120 whether or not the current average value BFt is same as the initial value BF0 (step S26). Namely, the blood flow volume calculating device 120 determines whether or not the average value BFt which is calculated as the current average value of the blood flow volume of the living body 900 is same as the initial value BF0 which is calculated as the blood flow volume when the artificial dialysis starts, namely, whether or not the blood flow volume of the living body 900 does not vary after the artificial dialysis starts and remains in the initial value BF0. In other words, in the case where the blood flow volume does not vary, the blood flow volume calculating device 120 determines whether the blood flow volume remains in the initial value BF0 which is a value when the artificial dialysis starts or whether the blood flow volume varies after the artificial dialysis starts and remains in a value which is different from the initial value BF0.

If it is determined that the current average value BFt is same as the initial value BF0 (step S26: YES), the process in the step S21 is performed again.

If it is determined that the current average value BFt is not same as (i.e. is different from) the initial value BF0 (step S26: NO), a value of a blood flow volume saturation flag BF2 is set to "1" (step S27). Incidentally, the blood flow volume saturation flag BF2 is a storing device for storing a determination result obtained by determining whether or not the current average value BFt is same as the initial value BF0, and an initial value of the blood flow volume saturation flag BF2 is set to "0" which indicates that the current average value BFt is same as the initial value BF0.

As described above, in the blood flow volume calculation process, the blood flow volume (specifically, the average value of the blood flow volume during the predetermined period) is calculated for each predetermined period based on the blood flow waveform which is outputted from the blood flow waveform output apparatus 800 after the artificial dialysis starts, it is determined whether the calculated blood flow volume varies and the determination result is recorded into the blood flow volume variation flag BF1 and the blood flow volume saturation flag BF2.

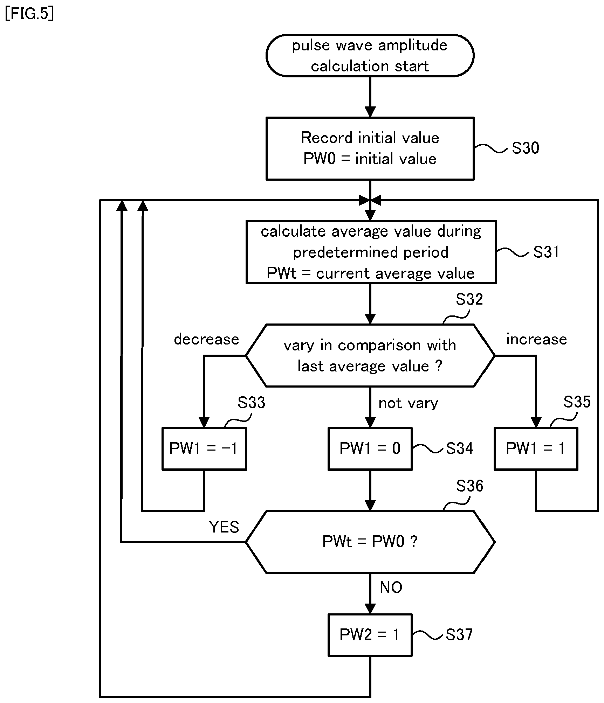

FIG. 5 is a flowchart illustrating a flow of the pulse wave amplitude calculation process in the blood pressure decrease prediction apparatus 100.

In FIG. 5, in the pulse wave amplitude calculation process, firstly, an initial value of the pulse wave amplitude is recorded by the pulse wave amplitude calculating device 130 (step S30). Namely, the pulse wave amplitude calculating device 130 calculates the pulse wave amplitude based on the blood flow waveform which is inputted from the blood flow waveform output apparatus 800 when the artificial dialysis starts, and records, as the initial value PW0 of the pulse wave amplitude, the calculated pulse wave amplitude into a memory for example.

Next, a current average value PWt of the pulse wave amplitude is calculated by the pulse wave amplitude calculating device 130 (step S31). Namely, the pulse wave amplitude calculating device 130 calculates the average value of the pulse wave amplitude during a predetermined period including a current time point based on the blood flow waveform which is inputted from the blood flow waveform output apparatus 800, and records, as the current average value PWt, the calculated average value into the memory for example.

Next, it is determined by the pulse wave amplitude calculating device 130 whether or not the current average value PWt varies in comparison with the last average value, more specifically, whether or not the current average value PWt "decreases", "increases" or "does not vary" in comparison with the last average value (step S32). Namely, the pulse wave amplitude calculating device 130 calculates the average value PWt of the pulse wave amplitude for each predetermined period, determines that the pulse wave amplitude decreases if the current average value PWt is smaller than the last average value PWt, determines that the pulse wave amplitude increases if the current average value PWt is larger than the last average value PWt, and determines that the pulse wave amplitude does not vary if the current average value PWt is same as the last average value PWt.

If it is determined that the pulse wave amplitude decreases (step S32: "decrease"), a value of a pulse wave amplitude variation flag PW1 is set to "-1" (step S33), if it is determined that the pulse wave amplitude does not vary (step S32: "not vary"), the value of the pulse wave amplitude variation flag PW1 is set to "0" (step S34), and if it is determined that the pulse wave amplitude increases (step S32: "increase"), the value of the pulse wave amplitude variation flag PW1 is set to "1" (step S35). Incidentally, the pulse wave amplitude variation flag PW1 is a storing device for storing a determination result obtained by determining that the current average value of the blood flow volume "decreases", "increases" or "does not vary" in comparison with the last average value.

After the value of the pulse wave amplitude variation flag PW1 is set to "-1" (step S33), the process in the step S31 is performed again.

After the value of the pulse wave amplitude variation flag PW1 is set to "1" (step S35), the process in the step S31 is performed again.

After the value of the pulse wave amplitude variation flag PW1 is set to "0" (step S34), it is determined by the pulse wave amplitude calculating device 130 whether or not the current average value PWt is same as the initial value PW0 (step S36). Namely, the pulse wave amplitude calculating device 130 determines whether or not the average value PWt which is calculated as the current average value of the pulse wave amplitude of the living body 900 is same as the initial value PW0 which is calculated as the pulse wave amplitude when the artificial dialysis starts, namely, whether or not the pulse wave amplitude of the living body 900 does not vary after the artificial dialysis starts and remains in the initial value PW0. In other words, in the case where the pulse wave amplitude does not vary, the pulse wave amplitude calculating device 130 determines whether the pulse wave amplitude remains in the initial value PW0 which is a value when the artificial dialysis starts or whether the pulse wave amplitude varies after the artificial dialysis starts and remains in a value which is different from the initial value PW0.

If it is determined that the current average value PWt is same as the initial value PW0 (step S36: YES), the process in the step S31 is performed again.

If it is determined that the current average value PWt is not same as (i.e. is different from) the initial value PW0 (step S36: NO), a value of a pulse wave amplitude saturation flag PW2 is set to "1" (step S37). Incidentally, the pulse wave amplitude saturation flag PW2 is a storing device for storing a determination result obtained by determining whether or not the current average value PWt is same as the initial value PW0, and an initial value of the pulse wave amplitude saturation flag PW2 is set to "0" which indicates that the current average value PWt is same as the initial value PW0.

As described above, in the pulse wave amplitude calculation process, the pulse wave amplitude (specifically, the average value of the pulse wave amplitude during the predetermined period) is calculated for each predetermined period based on the blood flow waveform which is outputted from the blood flow waveform output apparatus 800 after the artificial dialysis starts, it is determined whether the calculated pulse wave amplitude varies and the determination result is recorded into the pulse wave amplitude variation flag PW1 and the pulse wave amplitude saturation flag PW2.

Again in FIG. 1, the blood pressure decrease predicting device 140 is provided with the risk level determining device 150 and predicts the decrease of the blood pressure of the living body 900.

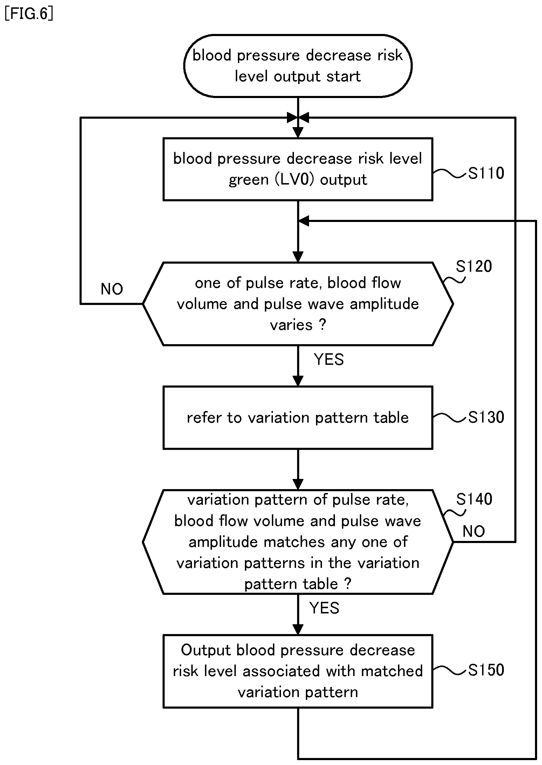

The risk level determining device 150 determines a blood pressure decrease risk level which indicates a risk for an occurrence of the decrease of the blood pressure of the living body 900 (i.e. indicates a possibility for the occurrence of the decrease of the blood pressure), based on the result of the above pulse rate calculation process, the above blood flow volume calculation process and the above pulse wave amplitude calculation process. In this embodiment, the blood pressure decrease risk level is determined to be one of the "LV0", "LV1", "LV2" and "LV3". Incidentally, a method of determining the blood pressure decrease risk level will be explained below in detail with reference to FIG. 6 to FIG. 8.

The outputting device 160 is an outputting device for outwardly outputting the blood pressure decrease risk level which is determined by the risk level determining device 150, and displays any one color of green color, yellow color, orange color and red color, in accordance with the blood pressure decrease risk level which is determined by the risk level determining device 150. Specifically, the outputting device 160 displays the green color if the blood pressure decrease risk level is "LV0", displays the yellow color if the blood pressure decrease risk level is "LV1", displays the orange color if the blood pressure decrease risk level is "LV2", and displays the red color if the blood pressure decrease risk level is "LV3". Namely, the outputting device 160 outwardly outputs, as the color, the blood pressure decrease risk level.

Next, the operation of the above configured blood pressure decrease prediction apparatus 100 will be explained with reference to FIG. 6 to FIG. 8.

FIG. 6 is a flowchart illustrating a flow of determination process of the blood pressure decrease risk level in the blood pressure decrease prediction apparatus 100.

In FIG. 6, when the blood pressure decrease prediction apparatus 100 operates, the green color is displayed by the outputting device 160 (step S110). Namely, the risk level determining device 150 sets, as an initial value, the blood pressure risk level to "LV0" which indicates the risk of the decrease of the blood pressure is very few or very low, and the outputting device 160 outwardly displays the green color in accordance with the set blood pressure decrease risk level (i.e. "LV0"). Incidentally, when the blood pressure decrease prediction apparatus 100 operates, the above described pulse rate calculation process, the above described blood flow volume calculation process and the above described pulse wave amplitude calculation process are repeatedly performed.

Next, it is determined (judged) by the risk level determining device 150 whether or not any one of the pulse rate, the blood flow volume and the pulse wave amplitude varies (step S120). Namely, the risk level determining device 150 determines whether or not any one of the pulse rate which is calculated by the pulse rate calculation process, the blood flow volume which is calculated by the blood flow volume calculation process and the pulse wave amplitude which is calculated by the pulse wave amplitude calculation process decreases or increases. Specifically, the risk level determining device 150 determines whether or not any one of the above described pulse rate variation flag HR1, the above described blood flow volume variation flag BF1 and the above described pulse wave amplitude variation flag PW1 is different from "0" (i.e. is "1" or "-1").

If it is determined that all of the pulse rate, the blood flow volume and the pulse wave amplitude do not vary (step S120: NO), the process in the step S110 is performed again. Namely, the risk level determining device 150 maintains the blood pressure decrease risk level as it remains in "LV0".

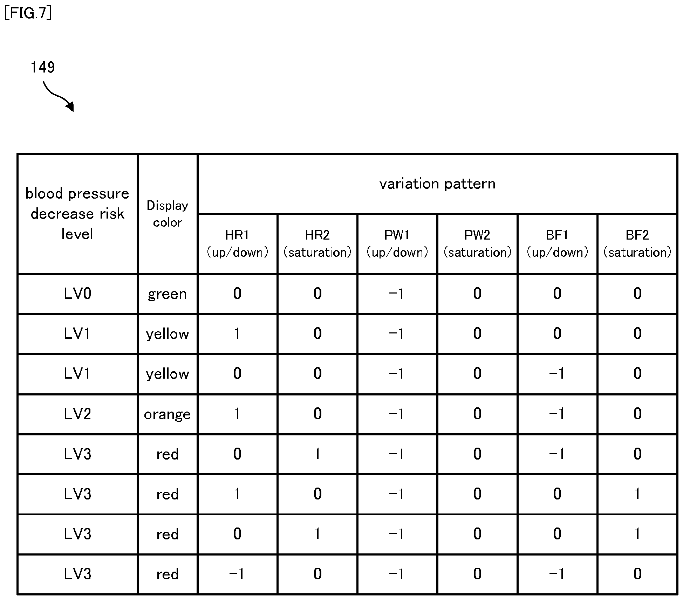

If it is determined that any one of the pulse rate, the blood flow volume and the pulse wave amplitude varies (step S120: YES), the blood pressure decrease risk level is determined by referring to a variation pattern table, and the color corresponding to the determined blood pressure decrease risk level is outwardly displayed (step S130, S140 and S150). Specifically, the risk level determining device 150 determines the blood pressure decrease risk level by referring to the variation pattern table 149 illustrated in FIG. 7. Incidentally, the variation pattern table 149 is one example of the "risk level determination table" of the present invention.

FIG. 7 is a conceptual diagram conceptually illustrating the variation pattern table 149 in the blood pressure decrease prediction apparatus 100.

In FIG. 7, the variation pattern table 149 is a look-up table which is for determining the blood pressure decrease risk level and which preliminarily stores combinations of applicable values of each of the pulse rate variation flag HR1, the pulse rate saturation flag HR2, the pulse wave amplitude variation flag PW1, the pulse wave amplitude saturation flag PW2, the blood flow volume variation flag BF1 and the blood flow volume saturation flag BF2, and the blood pressure decrease risk levels respectively corresponding to these combinations. In other words, the variation pattern table 149 is a look-up table in which the combinations of the variations of each of the pulse rate, the blood flow volume and the pulse wave amplitude are respectively associated with the risk levels which is anticipated when the respective combination occurs.

Specifically, in the variation pattern table 149, "LVO" which indicates that the risk of the decrease of the blood pressure is very few or very low is stored as the blood pressure decrease risk level corresponding to the combination (hereinafter, this combination is referred to as "0th variation patter") of the pulse rate variation flag HR1 being "0", the pulse rate saturation flag HR2 being "0", the pulse wave amplitude variation flag PW1 being "-1", the pulse wave amplitude saturation flag PW2 being "0", the blood flow volume variation flag BF1 being "0" and the blood flow volume saturation flag BF2 being "0". Moreover, in the variation pattern table 149, "LV1" which indicates that the risk of the decrease of the blood pressure is higher than "LV0" is stored as the blood pressure decrease risk level corresponding to the combination (hereinafter, this combination is referred to as "first variation patter") of the pulse rate variation flag HR1 being "1", the pulse rate saturation flag HR2 being "0", the pulse wave amplitude variation flag PW1 being "-1", the pulse wave amplitude saturation flag PW2 being "0", the blood flow volume variation flag BF1 being "0" and the blood flow volume saturation flag BF2 being "0". Moreover, in the variation pattern table 149, "LV1" is stored as the blood pressure decrease risk level corresponding to the combination (hereinafter, this combination is referred to as "second variation patter") of the pulse rate variation flag HR1 being "0", the pulse rate saturation flag HR2 being "0", the pulse wave amplitude variation flag PW1 being " - 1", the pulse wave amplitude saturation flag PW2 being "0", the blood flow volume variation flag BF1 being "-1" and the blood flow volume saturation flag BF2 being "0". Moreover, in the variation pattern table 149, "LV2" which indicates that the risk of the decrease of the blood pressure is higher than "LV1" is stored as the blood pressure decrease risk level corresponding to the combination (hereinafter, this combination is referred to as "third variation patter") of the pulse rate variation flag HR1 being "1", the pulse rate saturation flag HR2 being "0", the pulse wave amplitude variation flag PW1 being "-1", the pulse wave amplitude saturation flag PW2 being "0", the blood flow volume variation flag BF1 being "-1" and the blood flow volume saturation flag BF2 being "0". Moreover, in the variation pattern table 149, "LV3" which indicates that the risk of the decrease of the blood pressure is higher than "LV2" is stored as the blood pressure decrease risk level corresponding to the combination (hereinafter, this combination is referred to as "fourth variation patter") of the pulse rate variation flag HR1 being "0", the pulse rate saturation flag HR2 being "1", the pulse wave amplitude variation flag PW1 being "-1", the pulse wave amplitude saturation flag PW2 being "0", the blood flow volume variation flag BF1 being "-1" and the blood flow volume saturation flag BF2 being "0". Moreover, in the variation pattern table 149, "LV3" is stored as the blood pressure decrease risk level corresponding to the combination (hereinafter, this combination is referred to as "fifth variation patter") of the pulse rate variation flag HR1 being "1", the pulse rate saturation flag HR2 being "0", the pulse wave amplitude variation flag PW1 being " - 1", the pulse wave amplitude saturation flag PW2 being "0", the blood flow volume variation flag BF1 being "0" and the blood flow volume saturation flag BF2 being "1". Moreover, in the variation pattern table 149, "LV3" is stored as the blood pressure decrease risk level corresponding to the combination (hereinafter, this combination is referred to as "sixth variation patter") of the pulse rate variation flag HR1 being "0", the pulse rate saturation flag HR2 being "1", the pulse wave amplitude variation flag PW1 being "-1", the pulse wave amplitude saturation flag PW2 being "0", the blood flow volume variation flag BF1 being "0" and the blood flow volume saturation flag BF2 being "1". Moreover, in the variation pattern table 149, "LV3" is stored as the blood pressure decrease risk level corresponding to the combination (hereinafter, this combination is referred to as "seventh variation patter") of the pulse rate variation flag HR1 being "-1", the pulse rate saturation flag HR2 being "0", the pulse wave amplitude variation flag PW1 being " - 1", the pulse wave amplitude saturation flag PW2 being "0", the blood flow volume variation flag BF1 being "-1" and the blood flow volume saturation flag BF2 being "0" .

Furthermore, in the variation pattern table 149, display colors which are to be respectively displayed in accordance with the blood pressure decrease risk levels are stored. Specifically, "green color" is stored as the display color corresponding to the blood pressure decrease risk level "LV0", "yellow color" is stored as the display color corresponding to the blood pressure decrease risk level "LV1", "orange color" is stored as the display color corresponding to the blood pressure decrease risk level "LV2" and "red color" is stored as the display color corresponding to the blood pressure decrease risk level "LV3".

The above described variation pattern table 149 can be preliminarily prepared by presuming, experimentally, experientially or by a simulation in advance, the risk for the occurrence of the decrease of the blood pressure in the case where the combination of the variation of the pulse rate, the blood flow volume and the pulse wave amplitude occurs.

In FIG. 6 and FIG. 7, the risk level determining device 150 determines whether the variation pattern of the pulse rate, the blood flow volume and the pulse wave amplitude (namely, the combination of the pulse rate variation flag HR1, the pulse rate saturation flag HR2, the pulse wave amplitude variation flag PW1, the pulse wave amplitude saturation flag PW2, the blood flow volume variation flag BF1 and the blood flow volume saturation flag BF2) matches any one of the variation patterns (namely, 0th to seventh variation patterns) in the variation pattern table 149 (step S140).

If it is determined that the variation pattern of the pulse rate, the blood flow volume and the pulse wave amplitude does not match all of the variation patterns in the variation pattern table 149 (step S140: NO), the process in the step S110 is performed again.

If it is determined that the variation pattern of the pulse rate, the blood flow volume and the pulse wave amplitude matches any one of the variation patterns in the variation pattern table 149 (step S140: YES), the display color corresponding to the blood pressure decrease risk level which is associated with the matched variation pattern is outputted (step S150). Namely, the blood pressure decrease risk level stored in association with the variation pattern, which matches the variation pattern of the current pulse rate, the current blood flow volume and the current pulse wave amplitude, from among the variation patterns in the variation pattern table 149 is determined as the current blood pressure decrease risk level by the risk level determining device 150, and the display color corresponding to the determined blood pressure decrease risk level is displayed by the outputting device 160.

As described above, in the present example, the risk level determining device 150 determines the blood pressure decrease risk level by referring to the variation pattern table 149, based on the variations of the current pulse rate, the current blood flow volume and the current pulse wave amplitude which are obtained by the pulse rate calculation process, the blood flow volume calculation process and the pulse wave amplitude calculation process. Therefore, according to the risk level determining device 150, it is possible to precociously predict the decrease of the blood pressure before the decrease of the blood pressure occurs based on the variation of each of the pulse rate, the blood flow volume and the pulse wave amplitude, and to appropriately determine the blood pressure decrease risk level which indicates the risk for occurrence of the decrease of the blood pressure. Incidentally, when the blood pressure decrease risk level is determined, next (new) risk level may be determined based on current risk level in addition to the variation of each of the pulse rate, the blood flow volume and the pulse wave amplitude. Namely, in the case where the current blood pressure decrease risk level is "LV0", if the variation of each of the pulse rate, the blood flow volume and the pulse wave amplitude matches the variation pattern (namely, any one of the fourth to seventh variation pattern) corresponding to the blood pressure decrease risk level "LV3" on the variation pattern table 149, the blood pressure decrease risk level may be determined to be "LV1" which is one step higher blood pressure decrease risk level than the current blood pressure decrease risk level. Namely, the blood pressure decrease risk level may be determined such that the blood pressure decrease risk level is escalated step by step (i.e. the blood pressure decrease risk level is escalated step by step in the order of "LV0", then "LV1", then "LV2" and then "LV3"). In other words, for example, in the case where the blood pressure risk level is "LV0" and the green color is outwardly displayed currently, if the variation of each of the pulse rate, the blood flow volume and the pulse wave amplitude matches the variation pattern (namely, any one of the fourth to seventh variation pattern) corresponding to the blood pressure decrease risk level "LV3" on the variation pattern table 149, the display color may be changed from the green color to the yellow color instead of instead of being changed from the green color to the red color.

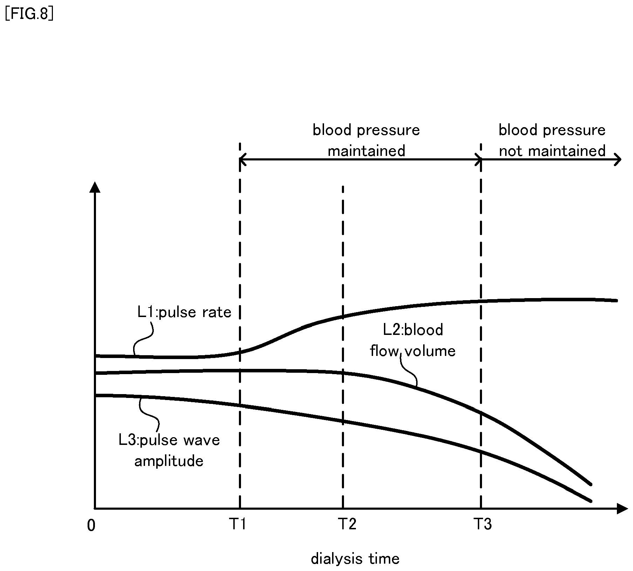

Here, FIG. 8 is a graph illustrating one example of a temporal variation of the pulse rate, the blood flow volume and the pulse wave amplitude of the living body 900 during the artificial dialysis. Incidentally, in FIG. 8, a horizontal axis is a time length (i.e. a dialysis time) that has elapsed since the artificial dialysis starts. Moreover, in FIG. 8, a solid line L1 is one example of a temporal variation of the pulse rate, a solid line L2 is one example of a temporal variation of the blood flow volume and a solid line L3 is one example of a temporal variation of the pulse wave amplitude.

In FIG. 8, the case is illustrated as one example where the circulating blood volume decreases due to the removal of water caused by the artificial dialysis and the pulse wave amplitude which may often vary depending on the variation of the one-time beat volume decreases along with the dialysis time. In the example of FIG. 8, after the artificial dialysis starts, the pulse rate of the living body 900 starts to increase at a timing when a time T1 has elapsed and becomes a constant value at a timing when a time T3 has elapsed because of saturation, the blood flow volume of the living body 900 starts to decrease at a timing when a time T2 which is longer than the time T1 and shorter than the time T3 has elapsed, and the pulse wave amplitude of the living body starts to decrease before the time T1 has elapsed and continues to decrease after the time T3 has elapsed.

The blood pressure is determined based on the product of a beat volume (i.e. the product of the one-time beat volume and the heart rate) and a peripheral vascular resistance (PVR). The living body 900 maintains the blood pressure by automatically adjusting the heart rate and the peripheral vascular resistance, if the blood pressure almost decreases. Namely, the heart rate and the peripheral vascular resistance often vary before the blood pressure decreases. The pulse rate corresponds to the heart rate, the blood flow volume often varies depending on the variation of the peripheral vascular resistance and the pulse wave amplitude varies depending on the one-time beat volume. In the example of FIG. 8, the pulse rate increases at the dialysis time T1. This indicates that the living body maintains the blood pressure by increasing the pulse rate. Moreover, in the example, of FIG. 8, the blood flow volume decreases at the dialysis time T2. This indicates that the living body maintains the blood pressure by increasing the peripheral vascular resistance. If the peripheral vascular resistance increases, the blood flow volume decreases. Namely, in the example of FIG. 8, a period from the dialysis time T1 to the dialysis time T2 is a stage (hereinafter, it is referred to as "a first blood pressure maintenance stage" as occasion demand) at which the living body maintains the blood pressure by increasing the pulse rate and a period from the dialysis time T2 to the dialysis time T3 is a stage (hereinafter, it is referred to as "a second blood pressure maintenance stage" as occasion demand) at which the living body maintains the blood pressure by increasing the peripheral vascular resistance. Moreover, a period after the dialysis time T3 is a period when the blood pressure cannot be maintained by varying the pulse rate and the peripheral vascular resistance, namely, a stage (hereinafter, it is referred to as "blood pressure maintenance impossible stage" as occasion demand) at which the decrease of the blood pressure occurs. As illustrated in FIG. 8, before the decrease of the blood pressure occurs, the pulse rate, the blood flow volume and the pulse wave amplitude vary depending on the degree for the possibility of the occurrence of the decrease of the blood pressure.

Therefore, in the blood pressure decrease prediction apparatus 100 of the present example, the blood pressure decrease risk level is determined by the risk level determining device 150 based on the variation pattern of the pulse rate, the blood flow volume and the pulse wave amplitude which are obtained from the blood flow waveform. Therefore, it is possible to precociously predict the decrease of the blood pressure before the decrease of the blood pressure occurs and to appropriately determine the blood pressure decrease risk level which indicates the risk of the occurrence of the decrease of the blood pressure. Thus, it is possible to inform the living body 900 who is the patient and an involved person who is a doctor or a nurse of the risk of the decrease of the blood pressure, by outwardly outputting the blood pressure decrease risk level determined by the risk level determining device 150. As a result, it is possible to reduce or prevent a delay of a treatment for the living body 900 whose blood pressure is predicted to decrease (i.e. it is possible to precociously treat the living body 900 who has the risk of the decrease of the blood pressure).

Incidentally, the blood pressure decrease prediction apparatus 100 is configured such that the variation pattern table 149 which is above described with reference to FIG. 7 can be re-written by a user (for example, the living body 900 who is the patient and the involved person who is the doctor or the nurse). Thus, it is possible to determine the blood pressure decrease risk level more appropriately (i.e. it is possible to precociously predict the decrease of the blood pressure more accurately).

Moreover, although the present example uses the example in which four levels, i.e. "LV0", "LV1", "LV2" and "LV3", are set, the number of the blood pressure decrease risk level may be increased by subdividing each level based on a variation rate (i.e. a variation volume per a predetermined time) of each of the pulse rate, the blood flow volume and the pulse wave amplitude. Moreover, the blood pressure decrease risk level may be set depending on whether the status of the living body 900 is the above first or second blood pressure maintenance stage.

Furthermore, especially in the present example, there is such an advantageous effect that it is possible to mostly or completely eliminate the burden on the living body 900, in comparison with the case where the blood pressure is measured by using the cuff or the electrode for example, because the pulse rate and the blood flow volume are calculated based on the blood flow waveform inputted from the blood flow waveform output apparatus 800 which is the laser blood flow meter.

In addition, in the present example, as described above, the outputting device 160 outwardly display any one color of the green color, the yellow color, the orange color and the red color, depending on the blood pressure decrease risk level determined by the risk level determining device 150. Therefore, it is possible for the living body 900 who is the patient and the involved person who is the doctor or the nurse to intuitively recognize the blood pressure decrease risk level. Incidentally, the outputting device 160 may be configured to output number(s), character(s), sentence(s), diagram(s), sign(s), sound or the like which depends on the blood pressure decrease risk level determined by the risk level determining device 150. Moreover, the outputting device 160 may make an external equipment such as a blood pressure meter automatically operate, by sending a signal to the external equipment such as the blood pressure meter depending on the blood pressure decrease risk level determined by the risk level determining device 150.

Incidentally, the blood pressure decrease prediction apparatus 100 may be configured to display a graph which simultaneously illustrate the variation of each of the pulse rate calculated by the pulse rate calculating device 110, the blood flow volume calculated by the blood flow volume calculating device 120 and the pulse wave amplitude calculated by the pulse wave amplitude calculating device 130. In this case, the living body 900 who is the patient and the involved person who is the doctor or the nurse can intuitively recognize the risk for occurrence of the decrease of the blood pressure, and it is practically very usable.

Moreover, the blood pressure decrease prediction apparatus 100 may not necessarily use the risk level determination table.

Moreover, the risk level determining device 150 may be configured to determine the blood pressure decrease risk level based on the dialysis time in addition to the variation pattern of the pulse rate, the blood flow volume and the pulse wave amplitude (i.e. the combination of the variation of each of the pulse rate, the blood flow volume and the pulse wave amplitude). In this case, it is possible to determine the blood pressure decrease risk level more appropriately.

Moreover, the risk level determining device 150 may be configured to calculate a correlation value between the blood flow volume and the pulse wave amplitude and to determine the blood pressure decrease risk level based on the calculated correlation value. For example, the risk level determining device 150 may determine that the blood pressure decrease risk level is a relatively high level if the blood flow volume and the pulse wave amplitude decreases with being correlated (i.e. the correlation value between the blood flow volume and the pulse wave amplitude is more than a predetermined value, and the blood flow volume and the pulse wave amplitude decrease). In this case, it is possible to determine the blood pressure decrease risk level more appropriately.

Moreover, the risk level determining device 150 may be configured to determine the blood pressure decrease risk level based on a deviation of an average of each of the pulse rate, the blood flow volume and the pulse wave amplitude. In this case, it is possible to determine the blood pressure decrease risk level more appropriately.

Moreover, the blood pressure decrease prediction apparatus 100 may be configured to determine the blood pressure decrease risk level based on other information related to the decrease of the blood pressure which is obtained from the blood flow waveform in addition to the pulse rate, the blood flow volume and the pulse wave amplitude. In this case, it is possible to determine the blood pressure decrease risk level more appropriately. In this case, the blood pressure decrease prediction apparatus 100 may determine the blood pressure decrease risk level based on information related to the decrease of the blood pressure of the patient who received the artificial dialysis in the past.

Moreover, the blood pressure decrease prediction apparatus 100 may be configured to ask the living body 900 who is the patient and the involved person who is the doctor or the nurse to measure the blood pressure of the living body 900 by using the blood pressure meter, depending on the blood pressure decrease risk level determined by the risk level determining device 150.

### <Modified Example>

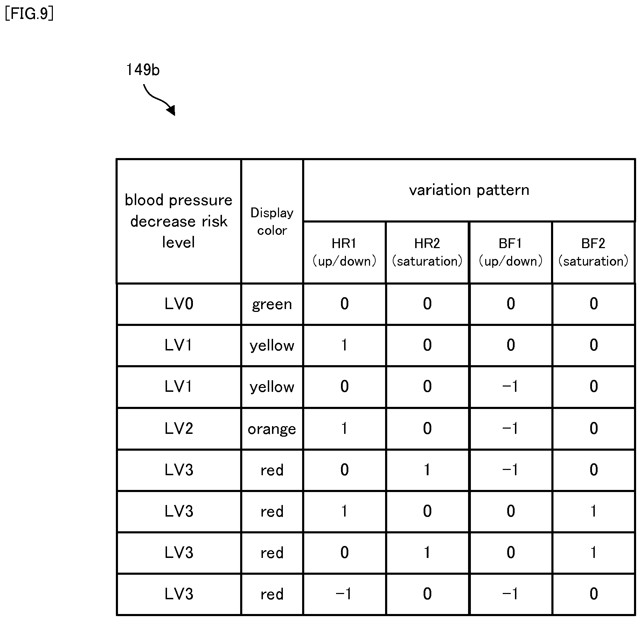

Although the above described embodiment uses the example in which the risk level determining device 150 determines the blood pressure decrease risk level by referring to the variation pattern table 149 illustrated in FIG. 7 based on the result of the pulse rate calculation process, the blood flow volume calculation process and the pulse wave amplitude calculation process, the risk level determining device 150 may determine the blood pressure decrease risk level by referring to the variation pattern table 149b illustrated in FIG. 9 based on the result of the pulse rate calculation process and the blood flow volume calculation process.

FIG. 9 is a conceptual diagram conceptually illustrating a variation pattern table 149b in a modified example.

In FIG. 9, the variation pattern table 149b is a look-up table which is for determining the blood pressure decrease risk level and which preliminarily stores combinations of applicable values of each of the pulse rate variation flag HR1, the pulse rate saturation flag HR2, the blood flow volume variation flag BF1 and the blood flow volume saturation flag BF2, and the blood pressure decrease risk levels respectively corresponding to these combinations. In other words, the variation pattern table 149b is a look-up table in which the combinations of the variations of each of the pulse rate and the blood flow volume are respectively associated with the risk levels which is anticipated when the respective combination occurs.

The above described variation pattern table 149b can be preliminarily prepared by presuming, experimentally, experientially or by a simulation in advance, the risk for the occurrence of the decrease of the blood pressure in the case where the combination of the variation of the pulse rate and the blood flow volume occurs.

According to this modified example, it is possible to precociously predict the decrease of the blood pressure by referring to the variation pattern table 149b based on the variation of the pulse rate and the blood flow volume and to appropriately determine the blood pressure decrease risk level.

As described above, it is possible to precociously predict the decrease of the blood pressure of the living body 900 without causing the burden on the living body 900 and to appropriately determine the blood pressure decrease risk level which indicates the risk for the occurrence of the blood pressure. Furthermore, it is possible to inform the living body 900 who is the patient and the involved person who is the doctor or the nurse of the risk of the decrease of the blood pressure. As a result, it is possible to precociously treat the living body 900 who has the risk of the decrease of the blood pressure.

A blood pressure decrease prediction apparatus, which involves such changes, is also intended to be within the technical scope of the present disclosure. Moreover, the present disclosure can be applied to an apparatus which predicts symptom of dehydration of the living body. Namely, it is possible to determine level of the symptom of the dehydration based on the blood pressure decrease risk level.

### Description of Reference Signs

- 100: blood pressure decrease prediction apparatus
- 110: pulse rate calculating device
- 120: blood flow volume calculating device
- 130: pulse wave amplitude calculating device
- 140: blood pressure decrease predicting device
- 149: variation pattern table
- 150: risk level determining device
- 160: outputting device
- 800: blood flow waveform output apparatus
- 900: living body

## Claims

1. A blood pressure decrease prediction apparatus (100) which is configured to predict a decrease of a blood pressure of a living body (900),
the blood pressure decrease prediction apparatus comprising:
a pulse rate calculating device (110) which is configured to calculate a pulse rate of the living body, based on a blood flow waveform which represents a temporal variation of a blood flow volume of the living body and which is inputted from a blood flow waveform output apparatus; and
a blood flow volume calculating device (120) which is configured to calculate the blood flow volume of the living body, based on the blood flow waveform;
**characterized in that**
the blood pressure decrease prediction apparatus further comprises: a blood pressure decrease predicting device (140) which is configured to predict the decrease of the blood pressure, based on (i) a first variation flag (HR1, BF1) which is determined on the basis of whether a current average value (HRt, BFt) of each of the calculated pulse rate and the calculated blood flow volume decreases, increases or does not vary in comparison with the last average value (HRt, BFt) of each of the calculated pulse rate and the calculated blood flow volume and (ii) a first saturation flag (HR2, BF2) which is determined on the basis of whether the current average value (HRt, BFt) of each of the calculated pulse rate and the calculated blood flow volume is same as an initial value (HR0, BF0) of each of the calculated pulse rate and the calculated blood flow volume, wherein the current average value is a calculated average value during a predetermined period including a current time point, the initial value is a value recorded when a dialysis starts.

2. The blood pressure decrease prediction apparatus according to claim 1, wherein
the blood pressure decrease predicting device (140) comprises a risk level determining device (150) which is configured to determine a risk level which indicates a risk for an occurrence of the decrease of the blood pressure, based on the first variation flag and the first saturation flag (HR1, HR2, BF1, BF2).

3. The blood pressure decrease prediction apparatus (100) according to claim 2, wherein
the risk level determining device (150) determines the risk level by referring to a predetermined risk level determination table (149b) in which a combination of the first variation flag and the first saturation flag (HR1, HR2, BF1, BF2) is associated with the risk level.

4. The blood pressure decrease prediction apparatus (100) according to claim 1, wherein
the blood pressure decrease prediction apparatus further comprises a pulse wave amplitude calculating device (130) which is configured to calculate a pulse wave amplitude of the living body, based on the blood flow waveform,
the blood pressure decrease predicting device predicts the decrease of the blood pressure, based on (i) a second variation flag (PW1) which is determined on the basis of whether a current average value (PWt) of the calculated pulse wave amplitude decreases, increases or does not vary in comparison with the last average value (PWt) of the calculated pulse wave amplitude and (ii) a second saturation flag (PW2) which is determined on the basis of whether the current average value (PWt) of the calculated pulse wave amplitude is same as an initial value (PW0) of the calculated pulse wave amplitude in addition to the first variation flag and the first saturation flag (HR1, HR2, BF1, BF2).

5. The blood pressure decrease prediction apparatus (100) according to claim 4, wherein
the blood pressure decrease predicting device (140) comprises a risk level determining device (150) which is configured to determine a risk level which indicates a risk for an occurrence of the decrease of the blood pressure, based on the first to second variation flags and the first to second saturation flags (HR1, HR2, BF1, BF2, PW1, PW2).

6. The blood pressure decrease prediction apparatus (100) according to claim 5, wherein
the risk level determining device (150) determines the risk level by referring to a predetermined risk level determination table (149) in which a combination of the first to second variation flags and the first to second saturation flag (HR1, HR2, BF1, BF2, PW1, PW2) is associated with the risk level.

7. The blood pressure decrease prediction apparatus (100) according to any one of claims 1 to 3, wherein
the blood pressure decrease predicting device (140) determines whether or not one of the calculated pulse rate and the calculated blood flow volume changes, and predicts the decrease of the blood pressure based on the result of the determination.

8. The blood pressure decrease prediction apparatus (100) according to any one of claims 4 to 6, wherein
the blood pressure decrease predicting device (140) determines whether or not one of the calculated pulse rate, the calculated blood flow volume and the calculated pulse wave amplitude changes, and predicts the decrease of the blood pressure based on the result of the determination.

9. The blood pressure decrease prediction apparatus (100) according to claim 2 or 5, wherein
the blood pressure decrease prediction apparatus further comprises an outputting device (160) which is configured to outwardly output the risk level determined by the risk level determining device (150).

## Patentansprüche

1. Blutdrucksenkung-Vorhersagevorrichtung (100), welche eingerichtet ist, um eine Senkung eines Blutdrucks eines lebendigen Körpers (900) vorherzusagen,
die Blutdrucksenkung-Vorhersagevorrichtung aufweisend:
eine Pulsfrequenz-Berechnungsvorrichtung (110), welche eingerichtet ist, um eine Pulsfrequenz des lebendigen Körpers zu berechnen, basierend auf einer Blutflusswellenform, welche eine zeitliche Variation eines Blutflussvolumens des lebendigen Körpers repräsentiert und welche von einem Blutflusswellenform-Ausgabeapparat eingegeben wird; und
eine Blutflussvolumen-Berechnungsvorrichtung (120), welche eingerichtet ist, das Blutflussvolumen des lebendigen Körpers zu berechnen, basierend auf der Blutflusswellenform;
**dadurch gekennzeichnet dass**,
die Blutdrucksenkung-Vorhersagevorrichtung ferner aufweist:
einen Blutdrucksenkung-Vorhersageapparat (140), welcher eingerichtet ist, um die Senkung des Blutdrucks vorherzusagen, basierend auf (i) einem ersten Variationskennzeichen (HR1, BF1), welches auf der Grundlage ermittelt wird, ob ein jeweils aktueller Durchschnittswert (HRt, BFt) der berechneten Pulsfrequenz und des berechneten Blutflussvolumens im Vergleich mit dem jeweils vorigen Durchschnittswert (HRt, BFt) der berechneten Pulsfrequenz und des berechneten Blutflussvolumens sinkt, steigt oder nicht variiert und (ii) einem ersten Sättigungskennzeichen (HR2, BF2), welches auf der Grundlage ermittelt wird, ob der jeweils aktuelle Durchschnittswert (HRt, BFt) der berechneten Pulsfrequenz und des berechneten Blutflussvolumens gleich einem jeweils Anfangswert (HR0, BF0) der berechneten Pulsfrequenz und des berechneten Blutflussvolumens ist, wobei der aktuelle Durchschnittswert ein berechneter Durchschnittswert während einer vorbestimmten Periode aufweisend einen aktuellen Zeitpunkt ist, wobei der Anfangswert ein Wert ist, der aufgezeichnet wird, wenn eine Dialyse beginnt.

2. Blutdrucksenkung-Vorhersagevorrichtung gemäß Anspruch 1,
wobei der Blutdrucksenkung-Vorhersageapparat (140) eine Risikoniveau-Ermittlungsvorrichtung (150) aufweist, welche eingerichtet ist, ein Risikoniveau zu ermitteln, welches ein Risiko für ein Auftreten der Senkung des Blutdrucks angibt, basierend auf dem ersten Variationskennzeichen und dem ersten Sättigungskennzeichen (HR1, HR2, BF1, BF2).

3. Blutdrucksenkung-Vorhersagevorrichtung (100) gemäß Anspruch 2,
wobei die Risikoniveau-Ermittlungsvorrichtung (150) das Risikoniveau ermittelt, mittels Verweisens auf eine vorbestimmte Risikoniveau-Ermittlungstabelle (149b), in welcher eine Kombination des ersten Variationskennzeichens und des ersten Sättigungskennzeichens (HR1, HR2, BF1, BF2) dem Risikoniveau zugeordnet ist.

4. Blutdrucksenkung-Vorhersagevorrichtung (100) gemäß Anspruch 1,
wobei die Blutdrucksenkung-Vorhersagevorrichtung ferner eine Pulswellenamplituden-Berechnungsvorrichtung (130) aufweist, welche eingerichtet ist, eine Pulswellenamplitude des lebendigen Körpers zu berechnen, basierend auf der Blutflusswellenform,
wobei der Blutdrucksenkung-Vorhersageapparat die Senkung des Blutdrucks vorhersagt, basierend auf (i) einem zweiten Variationskennzeichen (PW1), welches auf der Grundlage ermittelt wird, ob ein aktueller Durchschnittswert (PWt) der berechneten Pulswellenamplitude im Vergleich mit dem vorigen Durchschnittswert (PWt) der berechneten Pulswellenamplitude sinkt, steigt oder nicht variiert und (ii) einem zweiten Sättigungskennzeichen (PW2), welches auf der Grundlage ermittelt wird, ob der aktuelle Durchschnittswert (PWt) der berechneten Pulswellenamplitude gleich einem Anfangswert (PW0) der berechneten Pulswellenamplitude ist, zusätzlich zu dem ersten Variationskennzeichen und dem ersten Sättigungskennzeichen (HR1, HR2, BF1, BF2).

5. Blutdrucksenkung-Vorhersagevorrichtung (100) gemäß Anspruch 4,
wobei der Blutdrucksenkung-Vorhersageapparat (140) eine Risikoniveau-Ermittlungsvorrichtung (150) aufweist, welche eingerichtet ist, ein Risikoniveau zu ermitteln, welches ein Risiko für ein Auftreten der Senkung des Blutdrucks angibt, basierend auf den ersten bis zweiten Variationskennzeichen und den ersten bis zweiten Sättigungskennzeichen (HR1, HR2, BF1, BF2, PW1, PW2).

6. Blutdrucksenkung-Vorhersagevorrichtung (100) gemäß Anspruch 5,
wobei die Risikoniveau-Ermittlungsvorrichtung (150) das Risikoniveau ermittelt, mittels Verweisens auf eine vorbestimmte Risikoniveau-Ermittlungstabelle (149), in welcher eine Kombination der ersten bis zweiten Variationskennzeichen und der ersten bis zweiten Sättigungskennzeichen (HR1, HR2, BF1, BF2, PW1, PW2) dem Risikoniveau zugeordnet ist.

7. Blutdrucksenkung-Vorhersagevorrichtung (100) gemäß einem der Ansprüche 1 bis 3,
wobei der Blutdrucksenkung-Vorhersageapparat (140) ermittelt, ob sich eine der berechneten Pulsfrequenz und des berechneten Blutflussvolumens ändert, und die Senkung des Blutdrucks vorhersagt, basierend auf dem Ergebnis der Ermittlung.

8. Blutdrucksenkung-Vorhersagevorrichtung (100) gemäß einem der Ansprüche 4 bis 6,
wobei der Blutdrucksenkung-Vorhersageapparat (140) ermittelt, ob sich eine der berechneten Pulsfrequenz, des berechneten Blutflussvolumens und der berechneten Pulswellenamplitude ändert, und die Senkung des Blutdrucks vorhersagt, basierend auf dem Ergebnis der Ermittlung.

9. Blutdrucksenkung-Vorhersagevorrichtung (100) gemäß Anspruch 2 oder 5,
wobei die Blutdrucksenkung-Vorhersagevorrichtung ferner eine Ausgabevorrichtung (160) aufweist, welche eingerichtet ist, das von der Risikoniveau-Ermittlungsvorrichtung (150) ermittelte Risikoniveau äußerlich auszugeben.

## Revendications

1. Appareil de prédiction de baisse de pression artérielle (100) qui est configuré pour prédire une baisse d'une pression artérielle d'un corps vivant (900),
l'appareil de prédiction de baisse de pression artérielle comprenant :
un dispositif de calcul de fréquence du pouls (110) qui est configuré pour calculer une fréquence de pouls du corps vivant, sur la base d'une forme d'onde de flux sanguin qui représente une variation temporelle d'un volume de flux sanguin du corps vivant et qui est fournie par un appareil de délivrance de forme d'onde de flux sanguin ; et
un dispositif de calcul de volume de flux sanguin (120) qui est configuré pour calculer le volume de flux sanguin du corps vivant, sur la base de la forme d'onde de flux sanguin ;
**caractérisé en ce que**
l'appareil de prédiction de baisse de pression artérielle comprend en outre :
un dispositif de prédiction de baisse de pression artérielle (140) qui est configuré pour prédire la baisse de la pression artérielle, sur la base (i) d'un premier repère de variation (HR1, BF1) qui est déterminé sur la base du fait qu'une valeur moyenne actuelle (HRt, BFt) de chacun de la fréquence de pouls calculée et du volume de flux sanguin calculé diminue, augmente ou ne varie pas en comparaison avec la dernière valeur moyenne (HRt, BFt) de chacun de la fréquence de pouls calculée et du volume de flux sanguin calculé, et (ii) d'un premier repère de saturation (HR2, BF2) qui est déterminé sur la base du fait que la valeur moyenne actuelle (HRt, BFt) de chacun de la fréquence de pouls calculée et du volume de flux sanguin calculé soit identique ou non à une valeur initiale (HR0, BF0) de chacun de la fréquence de pouls calculée et du volume de flux sanguin calculé, dans lequel la valeur moyenne actuelle est une valeur moyenne calculée pendant une période prédéterminée qui comprend un moment actuel, et la valeur initiale est une valeur enregistrée au début d'une dialyse.

2. Appareil de prédiction de baisse de pression artérielle selon la revendication 1, dans lequel
le dispositif de prédiction de baisse de pression artérielle (140) comprend un dispositif de détermination de niveau de risque (150) qui est configuré pour déterminer un niveau de risque qui indique un risque de survenance de la baisse de la pression artérielle, sur la base du premier repère de variation et du premier repère de saturation (HR1, HR2, BF1, BF2).

3. Appareil de prédiction de baisse de pression artérielle (100) selon la revendication 2, dans lequel
le dispositif de détermination de niveau de risque (150) détermine le niveau de risque en référence à un tableau de détermination de niveau de risque prédéterminé (149b) dans lequel une combinaison du premier repère de variation et du premier repère de saturation (HR1, HR2, BF1, BF2) est associée au niveau de risque.

4. Appareil de prédiction de baisse de pression artérielle (100) selon la revendication 1, dans lequel
l'appareil de prédiction de baisse de pression artérielle comprend en outre un dispositif de calcul d'amplitude d'onde d'impulsion (130) qui est configuré pour calculer une amplitude d'onde d'impulsion du corps vivant, sur la base de la forme d'onde de flux sanguin,
le dispositif de prédiction de baisse de pression artérielle prédit la baisse de la pression artérielle, sur la base (i) d'un second repère de variation (PW1) qui est déterminé sur la base du fait qu'une valeur moyenne actuelle (PWt) de l'amplitude d'onde d'impulsion calculée diminue, augmente ou ne varie pas en comparaison avec la dernière valeur moyenne (PWt) de l'amplitude d'onde d'impulsion calculée, et (ii) d'un second repère de saturation (PW2) qui est déterminé sur la base du fait que la valeur moyenne actuelle (PWt) de l'amplitude d'onde d'impulsion calculée soit identique ou non à une valeur initiale (PW0) de l'amplitude d'onde d'impulsion calculée en plus du premier repère de variation et du premier repère de saturation (HR1, HR2, BF1, BF2).

5. Appareil de prédiction de baisse de pression artérielle (100) selon la revendication 4, dans lequel
le dispositif de prédiction de baisse de pression artérielle (140) comprend un dispositif de détermination de niveau de risque (150) qui est configuré pour déterminer un niveau de risque qui indique un risque de survenance de la baisse de la pression artérielle, sur la base du premier au second repères de variation et du premier au second repères de saturation (HR1, HR2, BF1, BF2, PW1, PW2).

6. Appareil de prédiction de baisse de pression artérielle (100) selon la revendication 5, dans lequel
le dispositif de détermination de niveau de risque (150) détermine le niveau de risque en référence à un tableau de détermination de niveau de risque prédéterminé (149) dans lequel une combinaison du premier au second repères de variation et du premier au second repères de saturation (HR1, HR2, BF1, BF2, PW1, PW2) est associée au niveau de risque.

7. Appareil de prédiction de baisse de pression artérielle (100) selon l'une quelconque des revendications 1 à 3, dans lequel
le dispositif de prédiction de baisse de pression artérielle (140) détermine si l'un de la fréquence de pouls calculée et du volume de flux sanguin calculé change ou non, et prédit la baisse de la pression artérielle sur la base du résultat de la détermination.

8. Appareil de prédiction de baisse de pression artérielle (100) selon l'une quelconque des revendications 4 à 6, dans lequel
le dispositif de prédiction de baisse de pression artérielle (140) détermine si l'un de la fréquence de pouls calculée, du volume de flux sanguin calculé et de l'amplitude d'onde d'impulsion calculée change ou non, et prédit la baisse de la pression artérielle sur la base du résultat de la détermination.

9. Appareil de prédiction de baisse de pression artérielle (100) selon la revendication 2 ou 5, dans lequel
l'appareil de prédiction de baisse de pression artérielle comprend en outre un dispositif de délivrance (160) qui est configuré pour délivrer le niveau de risque déterminé par le dispositif de détermination de niveau de risque (150).
